# EUROPEAN PATENT SPECIFICATION

(11) **EP 1 928 317 B1**
(45) Date of publication and mention of the grant of the patent: **02.03.2011**
(21) Application number: 06794133.6
(22) Date of filing: 28.09.2006
(51) Int. Cl.: A61B 5/22, A63B 21/078, A63B 24/00, G01P 15/00

(54) **EVALUATION METHOD OF A SPORTS PERFORMANCE**
VERFAHREN ZUR BEWERTUNG EINER SPORTLICHEN LEISTUNG
METHODE D'EVALUATION D'UNE PERFORMANCE SPORTIVE

(30) Priority: 30.09.2005 FI 20050983
(43) Date of publication of application: 11.06.2008
(73) Proprietor: Oulun Seudun Ammattikorkeakoulu, 90250 Oulu (FI)
(72) Inventor: HANNULA, Manne, 90440 Kempele (FI)
(74) Representative: Määttä, Jukka Tapani
(86) International application number: PCT/FI2006/050416
(87) International publication number: WO 2007/036611

(56) References cited:
- JP-A- 2006 122 343
- US-A1- 2004 094 613
- US-A1- 2004 250 618
- US-B1- 6 183 365
- US-B1- 6 231 481
- US-B1- 6 231 481
- US-B1- 6 904 801

## Description

This invention relates to a evaluation method of a sports performance and to a device for evaluating a maximum result in sports performances associated in lifting weights, for example bench press.

Best known way of evaluating a maximal bench press result of a person is to perform the bench press movement by adding weights to a bar gradually. Maximum result is the result, that the person can yet lift. Further, another known way of evaluating a maximal bench press result of a person is to examine, how many repetitions a person can do by using weights clearly smaller than the assumed maximum, and to evaluate the possible maximum from this number of repetitions, based on experience. Further, a specific device for measuring the muscle strength with a measuring arrangement included in the device, is disclosed in patent publication DE 19733595.

The problem related to these known solutions is risk of injury, when using maximum weights in empirical determination of the maximum result, since using big weights has always its own risks, e.g. risk of muscle ruptures, additionally for instance with competing athletes making maximum performances requires both mental and physical resources before competition and thus the maximum result should not be evaluated this way close to the competition moment. Further, in evaluating the maximum, the valuation accuracy of maximum is poor, when using the number of repetitions achieved by using clearly smaller weights than the maximum weight, since the evaluation is typically based solely on experience and the dependence relation, that functions for one person, does not necessarily function for another person. In addition, also this definition method of maximum is heavy, because it requires the experimenter to perform as many repetitions he can to fatigue. Further, with the method described in patent publication DE 19733595, the maximum strength can only be evaluated by using the large device in question, not by using for instance an official weightlifting bar.

A device attached to wrist is known from application EP1366712, which device includes a motion sensor, keyboard and display. The device is used for measuring and evaluating the movements of the user, from which movements variables are calculated, which variables are shown on the display of the device. The device can e.g. be used for concluding, if the person carrying the device is walking or running. Energy comsumption during motion can also be calculated from the information. Application publication US 2004186378 describes a pulsemeter to be attached to wrist, which pulsemeter can remove the noise signals distorting the pulse measurement, which noise signals are caused by the movement of the person.

The object of the invention is to solve the problems associated with the evaluation of maximum of bench press or other corresponding lifting motion and to achieve a multipurpose evaluation method of sports performance as well as a device, with which the maximal weight lifting results of a person can be evaluated, by using weights clearly smaller than maximum. The invention reduces the risk for injury in testing the maximum of bench press and additionally increases the precision of evaluation, when compared to a method based on analysing the number of repetitions achieved with smaller weights, due to the especially accurate measurement method. Further, the device is to be suited for use with the conventional weightlifting bars, loose weights or devices, that the devotee in the sport tends to use, and not be limited to e.g. a large special device allowing only specific movements and equipped with measurement sensors, as in the case of reference publication DE 19733595.

The object is achieved with the invention, that is a wrist watch-like bracelet, including a sensitive acceleration sensor and keyboard for feeding the information, as well as a display for presenting the evaluated maximum result. The invention is used for evaluating the maximum result e.g. in bench press, by using two different weights clearly smaller than the maximum, as follows: the user enters the weight of the first weight in the bar in kilograms with the keyboard into the bracelet and performs the lifting motion such, that the bar lifting phase is done as fast as possible. Then the other weight is placed into the bar, the weight of which weight is entered in kilograms into the bracelet and the lifting motion is performed again as in connection with the first weight. The bracelet saves the maximum accelerations during both lifting motions in lifting plase of the bar. The bracelet examines the differences in maximum accelerations during both lifts with a special algorithm and evaluates the maximal weight lifting result of the experimenter in kilograms, by using the maximum accelerations and kilogram amounts of weights used in the lifts and finally shows the result on the display of the bracelet. The bracelet can be used in several lifting motions, e.g. biceps curl or squat, in addition to bench press.

More exactly, the measurement method for sports performance according to the invention is characterised with what is described in connection with the characterizing part of the independent claims.

Next the invention is described in further detail referring to the accompanying drawings, wherein:
figure 1 illustrates the use of the invention in bench press,
figure 2 illustrates the operating principle of the device according to the invention,
figure 3 illustrates the signal measured by the sensor of the invention in an example case, and
figure 4 illustrates the operating principle of the maximum result evaluation method.

Next the invention is described with help of some exemplary embodiments.

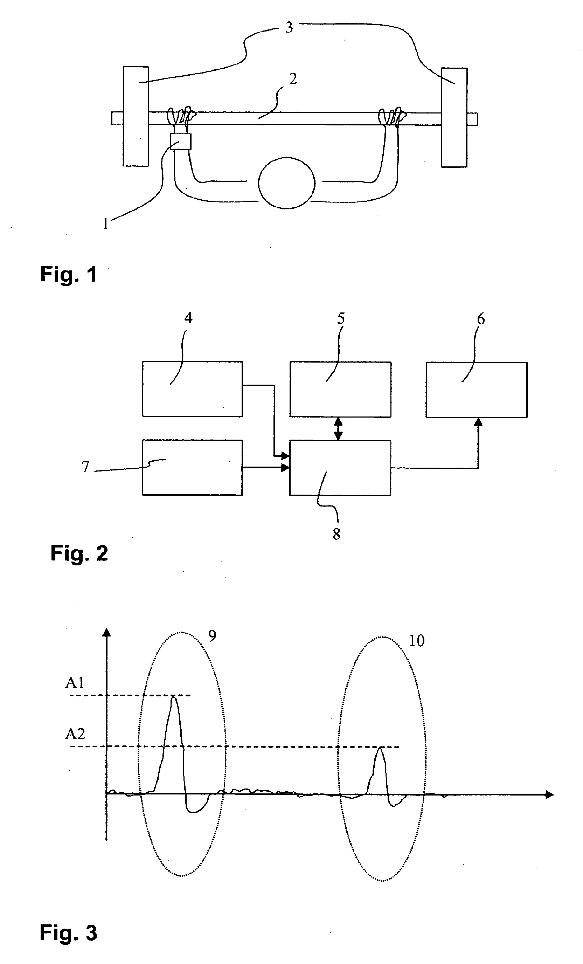

The invention is a bracelet 1, that is used in the example case illustrated in figure 1 to determine, how much weight a person can lift at maximum in bench press. As the person lifts the bar 2 and the weights 3 attached into it, the bracelet 1 moves to the same direction as the bar 2. The bracelet 1 consists more accurately of parts, that are illustrated as a diagram in figure 2. The bracelet consists of an acceleration sensor 4, a keyboard 7, a memory 5, a processor 8 and a display 6.

In an example case, in which it is assumed, that the bench press maximum of the person is well over hundred kilos, the invention functions as follows:

The person sets into the bar such a weight, that he can certainly lift, for instance 40 kilograms and enters this information into the bracelet with keyboard 7. The processor 8 of the bracelet saves the kilogram amount into the memory 5 of the device and starts recording the signal of the acceleration sensor 4. Next the person lifts the weight up as fast as he can. During the lift the processor 8 saves the signal of the acceleration sensor 4 to memory 5.

Next the person sets a second weight, bigger than the first weight, into the bar, for instance 70 kilograms and again enters the information into the memory 5 of the device with keyboard 7 and performs a new lifting motion by lifting the weight up as fast as he can. As with the first lift, the signal of the acceleration sensor 4 is saved to memory 5 of the bracelet.

The acceleration sensor signal during the two performed lifts is illustrated in figure 3, wherein two lifts are shown, the first lift 9 and the second lift 10. In the first lift 9 the largest acceleration has been A1 *m*/*s²* and in the second lift 10 the largest acceleration has been A2 *m*/*s²*.

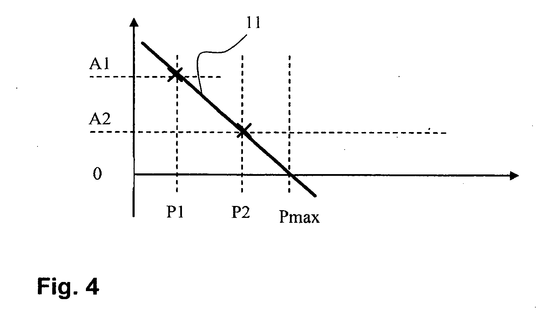

Next the processor 8 of the bracelet 1 starts to evaluate the maximum value of the bench press of the person, based on the information in the memory 5, by using the principle described in figure 4. Figure 4 includes a curve, horizontal axis of which depicts the lifted weight in kilograms and the vertical axis depicts the maximum acceleration of the lift in units *m*/*s².* Processor 8 performs a calculation, in which it first places the point of the first lift 9 into the coordinate system according to figure 4 to location (P1, A1) wherein P1 is the weight used in this lift and A1 is the maximum acceleration of this lift and the point of the second lift 10 to location (P2, A2) wherein P2 is the weight used in this lift and A2 is the maximum acceleration of this lift. Then the processor 8 defines a line 11, that travels through these points and examines, where this line reaches the value zero on the vertical axis and defines the reading Pₘₐₓ corresponding this point, which reading presents the estimated maximal result in the lifting motion in question and shows this result on the display 6.

This calculation is based on the idea, that when lifting the bar with a small weight, the maximum acceleration is large, but in the situation, where the lifting acceleration of the bar is zero, the person can no longer lift the bar, which means, that the weight is bigger than the person can lift. Thus the biggest weight the person can lift is roughly the point on the horizontal axis of figure 4, in which point the line 11 for the first time reaches the value zero.

Within the scope of the invention even solutions deviating from the aforedescribed can be considered. For instance the number of performed lifts can be more than two, when for instance three lifts can be used in calculating the maximum value Pₘₐₓ. Then adaption of a second order curve can be used in calculating the maximun value Pₘₐₓ.

The maximum value Pₘₐₓ can be estimated roughly also based merely on one measurement, by using a default value of the slope of line 11. Then the maximum value Pₘₐₓ can be estimated roughly based on only one measurement.

In addition, the maximum value Pₘₐₓ can be estimated from maximum accelerations and used weights, by using other than a linear line: the line 11 can be replaced for instance with a y=1/x -type curve or some other curve, that is fitted into the group of points formed of the maximum accelerations and used weights.

Further, a special algorithm can also be used in calculating the maximum performance, which algorithm uses, besides the signals of acceleration sensors, information of e.g. the height of the lifter, weight of the lifter and the age of the lifter e.g. such, that the algorithm observes also, that heavy persons typically have a large muscle mass, when probably the value of the actual maximum performance is a few kilograms greater than the value of lighter persons, even if the Pₘₐₓ-value calculated from the signals of acceleration sensors was the same for both persons.

Some preferred embodiments of the method and device according to the invention are described above. The invention is not however limited to the previously described embodiments. The inventional idea can be applied in several ways within the limits of the claims.

## Claims

1. An evaluation method for a maximal lifting result, in which a maximum acceleration created by a person is calculated by using a bracelet (1) attached to wrist, which bracelet (1) comprises:
- an acceleration sensor (4)
- a keyboard (7) for entering a lifting weight into the device
- a processor (8) and a memory (5) for performing and analysing the measurements, as well as
- a display (6) for presenting an estimated maximal lifting result of the user,
**characterised in that** in the method the estimate of the maximum lifting result that the person is capable of is evaluated by
- making at least two lifting motions by different non-maximum lifting weights with a maximum lifting speed
- measuring the maximal lifting accelerations of said at least two lifting motions, and
- calculating a value of the maximum lifting result by using a line (11) that is a tangent to the maximum acceleration values measured in said different lifting motions in a rectangular weight-acceleration coordinate system, and the mentioned line (11) intersects the weight axis at a point indicating the maximum lifting result.

2. An evaluation method for a maximal lifting result, in which a maximum acceleration created by a person is calculated by using a bracelet (1) attached to wrist, which bracelet (1) comprises:
- an acceleration sensor (4)
- a keyboard (7) for entering a lifting weight into the device
- a processor (8) and a memory (5) for performing and analysing the measurements, as well as
- a display (6) for presenting an estimated maximal llifting result of the user,
**characterised in that** in the method the value of the maximum lifting result that a person is capable of is evaluated with the help of one lifting motion made by a non-maximum lifting weight with maximum lifting speed and by using a default value of the slope of the line (11), when in a rectangular weight-acceleration coordinate system the value of the maximum performance is ascertained from a point, in which the line (11), calculated based on the measured maximum acceleration and the lifted weight as well as the assumed slope, intersects the weight axis of the coordinate system.

3. A bracelet (1) for defining a maximal lifting result, which bracelet comprises:
- an acceleration sensor (4)
- a keyboard (7) for entering a weight used in the lift into the device
- a processor (8) and a memory (5) for performing and analysing the measurements, as well as
- a display (6) for presenting an estimated maximum lifting result of the user,
**characterised in that** the bracelet further includes means for
- evaluating the value of the maximum lifting result by using maximal lifting accelerations measured in at least two lifting motions and made by two different non-maximum lifting weights with maximum lifting speeds used, and
- evaluating the value of the maximum lifting result by using a line (11) that is in a rectangular weight-acceleration coordinate system a tangent to said maximum acceleration values measured in said different lifting motions and the mentioned line (11) is arranged to intersect the weight axis at a point indicating the maximum lifting result.

4. A bracelet (1) for defining a maximal lifting result, which bracelet comprises:
- an acceleration sensor (4)
- a keyboard (7) for entering a weight used in the lift into the device
- a processor (8) and a memory (5) for performing and analysing the measurements, as well as
- a display (6) for presenting an estimated maximum lifting result of the user,
**characterised in that** the bracelet further includes means for evaluating the value of the maximum lifting result with the help of an acceleration measurement of one lifting motion made by a non-maximum lifting weight with maximum lifting speed and by using a default value of the slope of the line (11), when in a rectangular weight-acceleration coordinate system the value of the maximum lifting result is ascertained from a point, wherein the line (11), calculated based on the measured maximum acceleration and the lifted weight as well as the assumed slope, intersects the weight axis of the coordinate system.

## Patentansprüche

1. Ein Evaluationsverfahren für ein maximales Hebeergebnis, bei dem eine maximale Beschleunigung, die von einer Person erzeugt wird, durch Verwenden eines am Handgelenk befestigten Armbandes (1) berechnet wird, wobei das Armband (1) umfasst:
- einen Beschleunigungssensor (4),
- eine Tastatur (7), um ein zu hebendes Gewicht in die Einrichtung einzugeben,
- ein Prozessor (8) und einen Speicher (5), um die Messungen durchzuführen und zu analysieren, sowie
- eine Anzeige (6) zum Darstellen eines abgeschätzten maximalen Hebeergebnisses des Benutzers,
**dadurch gekennzeichnet, dass** bei dem Verfahren die Abschätzung des maximalen Hebeergebnisses, zu dem die Person in der Lage ist, evaluiert wird durch
- Vornehmen von zumindest zwei Hebebewegungen mit unterschiedlichen nicht maximalen Hebegewichten bei einer maximalen Hebegeschwindigkeit,
- Messen der maximalen Beschleunigungen der zumindest zwei Hebebewegungen, und
- Berechnen eines Wertes des maximalen Hebeergebnisses durch Verwenden einer Linie (11), die in einem rechtwinkligen Gewicht-Beschleunigungs-Koordinatensystem eine Tangente an die maximalen Beschleunigungswerte ist, die bei den unterschiedlichen Hebebewegungen gemessen wurden, und wobei die erwähnte Linie (11) die Gewichtsachse an einem Punkt schneidet, der das maximale Hebeergebnis anzeigt.

2. Evaluationsverfahren für ein maximales Hebeergebnis, bei dem eine maximale Beschleunigung, die von einer Person erzeugt wird, durch Verwenden eines am Handgelenk befestigten Armbandes (1) berechnet wird, wobei das Armband (1) umfasst:
- einen Beschleunigungssensor (4),
- eine Tastatur (7), um ein zu hebendes Gewicht in die Einrichtung einzugeben,
- ein Prozessor (8) und einen Speicher (5), um die Messungen durchzuführen und zu analysieren, sowie
- eine Anzeige (6) zum Darstellen eines abgeschätzten maximalen Hebeergebnisses des Benutzers,
**dadurch gekennzeichnet, dass** bei dem Verfahren der Wert des maximalen Hebeergebnisses zu dem eine Person in der Lage ist, mit der Hilfe von einer Hebebewegung, die mit einem nicht maximalen Hebegewicht bei maximaler Hebegeschwindigkeit gemacht wurde, und durch Verwenden eines Standartwertes der Steigung einer Line (11), evaluiert wird, wenn in einem rechtwinkligen Gewicht-Beschleunigungs-Koordinatensystem, der Wert der maximalen Leistung von einem Punkt aus ermittelt wird, an dem die Linie (11), die basierend auf der gemessenen maximalen Beschleunigung und dem gehobenen Gewicht sowie der angenommen Steigung berechnet wurde, die Gewichtsachse des Koordinatensystems schneidet.

3. Armband (1) zum Festlegen eines maximalen Hebeergebnisses, wobei das Armband umfasst:
- einen Beschleunigungssensor (4)
- eine Tastatur (7), um ein zu hebendes Gewicht in die Einrichtung einzugeben,
- ein Prozessor (8) und einen Speicher (5), um die Messungen durchzuführen und zu analysieren, sowie
- eine Anzeige (6) zum Darstellen eines abgeschätzten maximalen Hebeergebnisses des Benutzers,
**dadurch gekennzeichnet, dass** das Armband ferner Mittel zum
- Evaluieren des Wertes des maximalen Hebeergebnisses durch Verwenden maximaler Hebebeschleunigungen, die bei zumindest zwei Hebebewegungen gemessen wurden, die mit zwei unterschiedlichen nicht maximalen Hebegewichten unter Verwendung maximaler Hebegeschwindigkeiten gemacht wurden, und zum
- Evaluieren des Wertes des maximalen Hebeergebnisses durch Verwenden einer Linie (11), die in einem rechtwinkligen Gewicht-Beschleunigungs-Koordinatensystem eine Tangente an die maximalen Beschleunigungswerte ist, die bei den unterschiedlichen Hebebewegungen gemessen wurde, und wobei die erwähnte Linie (11) angeordnet ist, um sich mit der Gewichtsachse an einem Punkt zu schneiden, der das maximale Hebeergebnis anzeigt, umfasst.

4. Armband (1) zum Festlegen eines maximalen Hebeergebnisses, wobei das Armband umfasst:
- einen Beschleunigungssensor (4),
- eine Tastatur (7), um ein zu hebendes Gewicht in die Einrichtung einzugeben,
- ein Prozessor (8) und einen Speicher (5), um die Messungen durchzuführen und zu analysieren, sowie
- eine Anzeige (6) zum Darstellen eines abgeschätzten maximalen Hebeergebnisses des Benutzers,
**dadurch gekennzeichnet, dass** das Armband ferner Mittel zum Evaluieren des Wertes des maximalen Hebeergebnisses mittels der Hilfe einer Beschleunigungsmessung einer Hebebewegung, die mit einem nicht maximalen Hebegewicht bei maximaler Hebegeschwindigkeit gemacht wurde, durch Verwenden eines Standartwertes für die Steigung der Linie (11) umfasst, wenn in einem rechtwinkligen Gewicht-Beschleunigungs-Koordinatensystem der Wert des maximalen Hebeergebnisses an einem Punkt ermittelt wird, an dem die Linie (11), die basierend auf der gemessenen maximalen Beschleunigung und dem gehobenen Gewicht sowie der angenommen Steigung berechnet wurde, die Gewichtsachse des Koordinatensystems schneidet.

## Revendications

1. Procédé d'évaluation pour un résultat de levée maximum, dans lequel une accélération maximum créée par une personne est calculée en utilisant un bracelet (1) fixé au poignet, lequel bracelet (1) comprend :
un capteur d'accélération (4) ;
un clavier (7) pour entrer un poids de levée dans le dispositif ;
un processeur (8) et une mémoire (5) pour réaliser et analyser les mesures, ainsi que :
un écran (6) pour présenter un résultat de levée maximum estimé de l'utilisateur, **caractérisé en ce que**, dans le procédé, l'estimation du résultat de levée maximum dont la personne est capable est évaluée par les étapes consistant à :
réaliser au moins deux mouvements de levée par des poids de levée non maximum différents avec une vitesse de levée maximum ;
mesurer les accélérations de levée maximum desdits au moins deux mouvements de levée, et
calculer une valeur du résultat de levée maximum en utilisant une ligne (11) qui est une tangente par rapport aux valeurs d'accélération maximum mesurées dans lesdits mouvements de levée différents dans un système de coordonnées rectangulaire de poids-accélération, la ligne (11) mentionnée coupe l'axe des poids en un point indiquant le résultat de levée maximum.

2. Procédé d'évaluation pour un résultat de levée maximum, dans lequel une accélération maximum créée par une personne, est calculée en utilisant un bracelet (1) fixé sur le poignet, lequel bracelet (1) comprend :
un capteur d'accélération (4) ;
un clavier (7) pour entrer un poids de levée dans le dispositif ;
un processeur (8) et une mémoire (5) pour réaliser et analyser les mesures, ainsi que :
un écran (6) pour présenter un résultat de levée maximum estimé de l'utilisateur, **caractérisé en ce que** dans le procédé, la valeur du résultat de levée maximum dont est capable une personne, est évaluée à l'aide d'un mouvement de levée réalisé par un poids de levée non maximum avec une vitesse de levée maximum, et en utilisant une valeur par défaut de l'inclinaison de la ligne (11), lorsque dans un système de coordonnées rectangulaire de poids-accélération, la valeur de la performance maximum est établie à partir d'un point, dans lequel la ligne (11), calculée en fonction de l'accélération maximum mesurée et du poids levé ainsi que l'inclinaison supposée, coupe l'axe des poids du système de coordonnées.

3. Bracelet (1) pour définir un résultat de levée maximum, lequel bracelet comprend :
un capteur d'accélération (4) ;
un clavier (7) pour entrer un poids utilisé pour la levée dans le dispositif ;
un processeur (8) et une mémoire (5) pour réaliser et analyser les mesures, ainsi que :
un écran (6) pour présenter un résultat de levée maximum estimé de l'utilisateur, **caractérisé en ce que** le bracelet comprend en outre des moyens pour :
évaluer la valeur du résultat de levée maximum en utilisant les accélérations de levée maximum mesurées dans au moins deux mouvements de levée et évaluer la valeur du résultat de levée maximum en utilisant des accélérations de levée maximum mesurées dans au moins deux mouvements de levée et réalisées par deux poids de levée non maximum différents avec les vitesses de levée maximum utilisées, et
évaluer la valeur du résultat de levée maximum en utilisant une ligne (11) qui est dans un système de coordonnées rectangulaire de poids-accélération, une tangente auxdites valeurs d'accélération maximum mesurées dans lesdits mouvements de levée différents et la ligne mentionnée (11) est agencée pour couper l'axe des poids en un point indiquant le résultat de levée maximum.

4. Bracelet (1) pour définir un résultat de levée maximum, lequel bracelet comprend :
un capteur d'accélération (4) ;
un clavier (7) pour entrer un poids utilisé pour la levée dans le dispositif ;
un processeur (8) et une mémoire (5) pour réaliser et analyser les mesures, ainsi que
un écran (6) pour présenter un résultat de levée maximum estimé de l'utilisateur, **caractérisé en ce que** le bracelet comprend en outre des moyens pour évaluer la valeur du résultat de levée maximum à l'aide d'une mesure d'accélération d'un mouvement de levée réalisé par un poids de levée non maximum avec une vitesse de levée maximum, et en utilisant une valeur par défaut de l'inclinaison de la ligne (11), lorsque dans un système de coordonnées rectangulaire de poids-accélération, la valeur du résultat de levée maximum est établie à partir d'un point, dans lequel la ligne (11), calculée en fonction de l'accélération maximum mesurée et du poids levé ainsi que de la pente supposée, coupe l'axe des poids du système de coordonnées.
